# EUROPEAN PATENT SPECIFICATION

(11) **EP 2 497 477 B1**
(45) Date of publication and mention of the grant of the patent: **07.08.2013**
(21) Application number: 11157405.9
(22) Date of filing: 09.03.2011
(51) Int. Cl.: A61K 31/52, A61K 36/06, A61P 31/00

(54) **Use of a Cunninghamella alcoholic extract as antimicrobial**
Verwendung eines alkoholischen Extrakts von Cunninghamella als antimikrobielles Agens
Utilisation d'un extrait alcoolique de Cunninghamella comme antimicrobien

(43) Date of publication of application: 12.09.2012
(73) Proprietor: King Saud University, 11421 Riyadh (SA)
(72) Inventor: Awaad, Amani S., 11495, Riyadh (SA); Soliman, Gamal A., 11495, Riyadh (SA); Aljaber, Nabila A., 11495, Riyadh (SA); Al-Hamad, Tahani A., 11495, Riyadh (SA)
(74) Representative: Goddar, Heinz J.

(56) References cited:
- GB-A- 2 152 042
- MONTESINOS M CARMEN ET AL: "Adenosine promotes wound healing and mediates angiogenesis in response to tissue injury via occupancy of A(2A) receptors.", THE AMERICAN JOURNAL OF PATHOLOGY JUN 2002 LNKD- PUBMED:12057906, vol. 160, no. 6, June 2002 (2002-06), pages 2009-2018, XP002654241, ISSN: 0002-9440
- CRONSTEIN BRUCE N: "Adenosine receptors and wound healing.", THESCIENTIFICWORLDJOURNAL 16 JAN 2004 LNKD- PUBMED:14755098, vol. 4, 16 January 2004 (2004-01-16), pages 1-8, XP009150802, ISSN: 1537-744X
- STYLIANOS FAKAS ET AL: "Lipids of Cunninghamella echinulata with emphasis to [gamma]-linolenic acid distribution among lipid classes", APPLIED MICROBIOLOGY AND BIOTECHNOLOGY, SPRINGER, BERLIN, DE, vol. 73, no. 3, 19 July 2006 (2006-07-19), pages 676-683, XP019458735, ISSN: 1432-0614, DOI: DOI:10.1007/S00253-006-0506-3
- FAKAS STYLIANOS ET AL: "Compositional shifts in lipid fractions during lipid turnover in Cunninghamella echinulata", ENZYME AND MICROBIAL TECHNOLOGY, vol. 40, no. 5, April 2007 (2007-04), pages 1321-1327, XP002654242, ISSN: 0141-0229
- Anonymous: "Section 14, infectious diseases" In: Anonymous: "The Merck manual of duiagnosis and therapy. Eighteenth edition", 2006, Merck Reserach Laboratories, Whitehouse Station, NJ, XP002654243, ISBN: 0911910182 pages 1406-1476, * the whole document *

## Description

The present invention relates to an alcoholic extract of fungi, a method for its preparation and use thereof.

The fungal kingdom includes many species with unique and unusual biochemical pathways. The products of these pathways include important pharmaceuticals such as penicillin, cyclosporine and statins; potent poisons including aflatoxins and trichothecenes; and some Janusfaced metabolites that are both toxic and pharmaceutically useful such as the ergot alkaloids. All of these natural products, along with low-molecular weight metabolites, are classified together as secondary metabolites, see Keller et al., Nature Reviews Microbiology, 3, 937-947 (2005). The fungal secondary metabolites have a wide range of chemical structure and biological activities. They are derived from many different intermediates by special enzymatic pathways encoded by a specific genus. The fungal secondary metabolites, or biochemical indicators of fungal development, are of great interest to humankind due to their pharmaceutical and/or toxic properties.

*Cunninghamella* is a genus of fungus in the Mucorales order, and the family Cunninghamellaceae is found in soil and plant material, particularly at Mediterranean and subtropical zones. Members of this genus are often used in studies investigating the metabolism of drugs, because these species metabolize a wide range of drugs in manners similar to mammalian enzyme systems, see Asha et al., Biotechnol. Adv. 27 (1): 16-29 (2009). The production of secondary metabolites by fungi is frequently associated with differentiation and sporulation and is effected by various kinds of environmental stress. Secondary metabolites occur as growth rate declines and during the stationery phase, see for example Trail et al., Microbiology, 141, 755-765 (1995).

Montesinos et al. American Journal of Pathology, 2002, 160 (6), 2009-2018, describes that adenosine promotes wound healing and mediates angiogenesis in response to tissue injury via occupancy of A_{2A} receptors.

Cronstein, The Scientific World Journal, 2004, 4, 1-8, reviews known effects of adenosine A_{2A} receptor occupancy on the cells involved in wound healing.

Fakas et al., Appl. Microbiol. Biotechnol. (2006), 73, 676-683, refers to lipids of *Cunning-hantella echinulata* with emphasis to γ-linolenic acid distribution among lipid classes.

Fakas et al., Enzyme and Microbial Technology, 2007, 40, 1321-1327, discloses compositional shifts in lipid fractions during lipid turnover in *Cunninghamella echinulata.*

GB 2 152 042 A refers to a process for preparing a lipid composition having a high γ-linolenic acid content by culturing mold fungi of the genus *Cunninghamella* in an aqueous nutrient culture medium having a relatively high concentration of a carbon source, and the lipid composition is recovered from the cultured mold fungi, and possibly saponified to liberate free acid.

A number of antibiotics is known in the art. Also known is, however, that the resistance to a known antibiotics is increasing.

Known antibiotics have many disadvantages on different body organs, especially on liver and kidney functions, i.e. safety of known antibiotics can be improved.

It is thus an object of the present invention to provide an antibiotic/antimicrobial agent which overcomes the difficulties and disadvantages of the prior art, especially disadvantages related to body organs, such as liver and kidney functions.

This object is achieved by the alcoholic extract for use in therapy as antibiotic and/or antimicrobial agent according to claim 1 and adenosine for use in therapy as antibiotic agent. Preferred embodiments result from the sub-claims.

Surprisingly it was found that according to the invention a new antibiotic/antimicrobial agent can be provided which shows sufficient antimicrobial activity with sub-chronic toxicity and which can be successfully utilized.

It was further found that some of the constituents of the inventive alcoholic extract could have been isolated and investigated regarding their capability of also acting as antimicrobial/antibiotic agent. In this regard it was surprisingly found that especially adenosin (2-(6-amino-9H-purin-9-yl)-5-(hydroxymethyl)oxolan-3,4-diol) shows surprising results especially antibiotic.

According to the invention is therefore an alcoholic extract of fungi of the genus *Cunninghamella* for treatment of antibiotic and/or antimicrobial diseases.

The inventive extract and/or the adenosine can be provided in liquid or solid form, for example as extract or solution, or can be formed into tablets, both for oral and topical application.

Additional advantages and features of the present invention can be taken from the following detailed description of preferred embodiments.

The antimicrobial activities in addition to acute and sub-chronic toxicity of methanol extracts of the three fungi (*C*. *blakeshleeana, C. elegans* and *C*. *homothallica*) and fractions of *C*. *elegans* are investigated. Described herein is an in vivo experimental evaluation of the wound healing effect of *C*. *elegans* extract and isolated compounds is given.

### Material and methods

### Fungal material

From the German Collection of Microorganisms and Cell Cultures (DMMZ) the investigated fungi, *Cunninghamella blakesleeana* (DSM 1906), *Cunninghamella homothallica* (DSM 1156) and *Cunninghamella elegans* (DSM 1908) were obtained. A direct inoculation method was used for sampling and isolation of fungal isolates. For isolation culturing, maintenance of stock cultures and experimental studies the following range of media were used: Czapek-Dox Agar (sucrose, 30 g; sodium nitrate, 2 g, potassium hydrogen orthophosphate, 1 g; potassium chloride, 0.5 g; magnesium sulphate, 0.5 g; ferrous sulphate, 0.002 g; agar, 20 G; distilled water, 1 1), malt extract agar (malt extract, 20 g; peptone, 1 g; glucose, 20 g; agar, 20 g; distilled water, 1 1), potato dextrose agar (potato extract, 4 g; glucose, 20 g; agar, 20 g; distilled water, 1 1), yeast extract sucrose agar (yeast extract, 20 g; sucrose, 150 g; agar, 20 g; distilled water, 1 1). The bacterial test organisms were cultured on nutrient agar (peptone, 5 g; beef extract, 3 g; sodium chloride, 3 g; agar, 20 g; distilled water, 1 1). A liquid medium was prepared by using the same ingredients without agar.

### Apparatus

For identification of isolated compounds, melting points were determined on a Kofler hot-stage apparatus (uncorrected); mass spectra (electrospray negative ion) were obtained on a micromass Quattro spectrometer dissolving the samples in acetonitrile. ¹H and ¹³C NMR spectra were recorded using external electronic referencing through the deuterium resonance frequency of the solvent, at 600.17 or 150.91 MHz, respectively, with a JEOL ECA 600 spectrometer fitted with an auto 5 mm X/H probe. Carbon atom types were detected in the ¹³CNMR spectrum by employing a combination of broad- and proton-decoupled and distortionless enhancement by polarization transfer (DEPT) experiments with 64 K data points over a spectrum width of 17, 605.6 MHz. [¹J_{C-H}] and ²J_{C-H} and ³J_{C-H}]. ¹H-¹³C correlations were established by using heteronuclear multiple quantum correlation (HMQC) and heteronuclear multiple bond connectivity (HMBC) pulse sequences, respectively. ¹H-¹H correlations were by double quantum filtered COSY.

### Extraction and isolation

The mycelia mat of *Cunninghamella elegans* (700 g) was harvested, washed with distilled water and then extracted by refluxing in boiling methanol (2 1) for 2 hours and filtered off. The mycelia residue was re-extracted again for three times. The combined filtrates were concentrated under reduced pressure at a temperature not exceeding 35°C. The obtained residue (67 g) was kept for investigation.

A part of the obtained residue of mycelia mat extract was diluted with distilled water (200 ml) and successively extracted using diethyl ether, chloroform, ethyl acetate and n-butanol. Each successive extract was dried over anhydrous sodium sulphate and concentrated using reduced pressure to obtain residues. All fungal successive extracts were chromatographically investigated on pre-coated silica gel GF plates using (a) chloroform/diethyl ether 50:50 (v/v), b) chloroform/methanol 96:4 (v/v) and c) ethylacetate/methanol/water 30:5:4 (v/v/v) as solvent systems. Both diethyl ether and chloroform extracts showed the same pattern on thin layer chromatography TLC (numbers and color of spots) and on the other hand both ethyl acetate and n-butanol extracts were similar to each other, therefore each similar extracts were collected together and symbolized as A1 and A2. Isolation of compounds from these extracts was carried out using extracts A1 and A2 separately.

Therefore, A1 was applied on a column packed with silica gel and eluted with ether gradually increased with chloroform. A number of fractions was obtained, the residue of each fraction was separately reapplied on columns packed with silica gel and eluted with chloroform gradually increased with methanol, from which compounds palmitic acid **1,** oleic acid **2,** stearic acid **3,** galic acid (3,4,5-trihydroxy-benzoic acid) **7** and 3-(methoxycarbonyl)-but-3-enoic acid **8** could be obtained. Optionally, these compounds can be further purified by applying on a column packed with sephadex LH 20 and eluted with methanol-water 1:1 (v/v).

Extract A2 was applied on a column packed with silica gel and eluted with ethyl acetate gradually increased with methanol. Three mean fractions were obtained. Each fraction was concentrated under reduced pressure, and the residue of each fraction group was then separately reapplied on columns packed with silica gel and eluted with ethyl acetate gradually increased with ethanol. Adenosine (2-(6-amino-9H-purine-9-yl)-5-(hydroxymethyl)oxolane-3,4-diol **4**, uridine (pyrimidine-2,4-dion,1-ribose) 5 and uracil (pyrimidine-2,5-dion) **6** were obtained.

The isolated compounds from Cunninghamella elegans are:

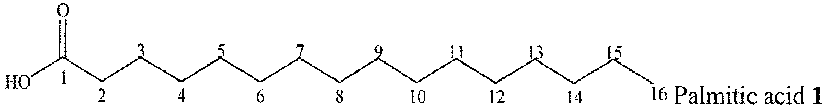

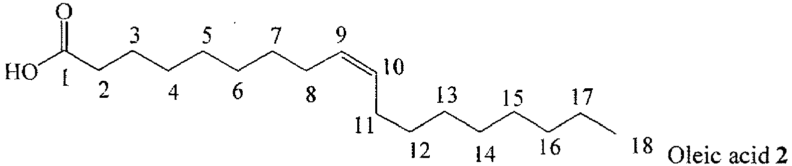

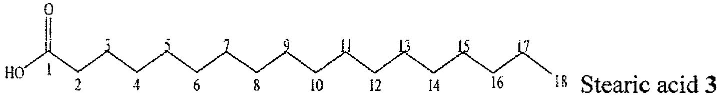

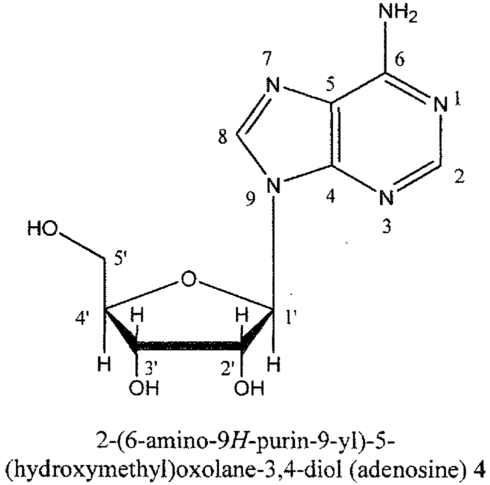

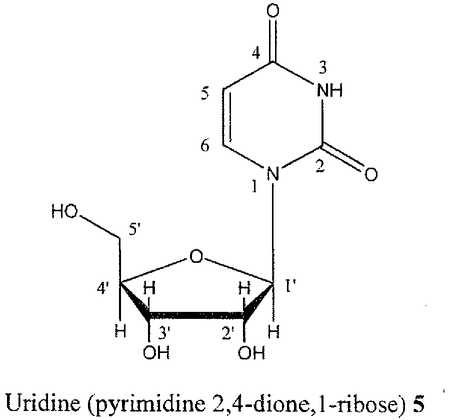

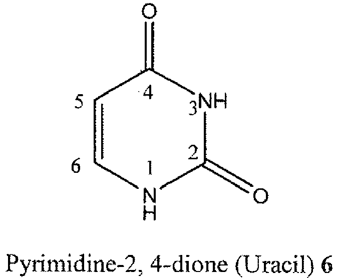

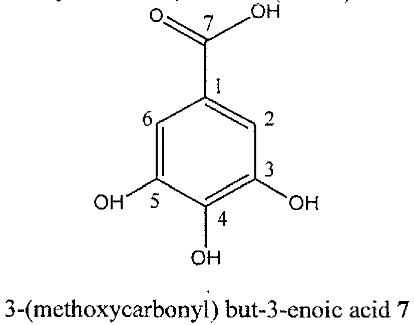

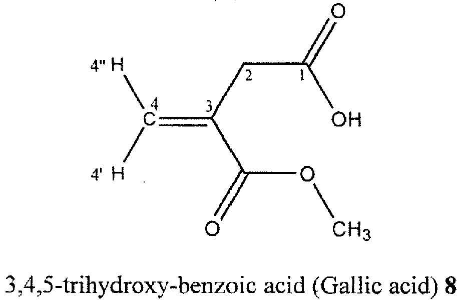

### Biological studies:

### 1. Antimicrobial activity

### Preparation of the extracts

The alcoholic extracts obtained were used for testing their antimicrobial activity. Each extract (0.5 g) was dissolved in 10 ml water. Same concentrations for the successive extracts (using diethyl ether, chloroform, ethyl acetate and n-butanol) were also prepared.

### Test organisms

The representatives of Gram negative bacteria, namely *Salmonella typhimuium* (RCMB 0104), *Escherichia coli* (RCMB 0103), *Pseudomonas aeruginosa* (RCMB 0102) and Gram positive bacteria, namely *Bacillus subtilis* (RCMB 0107), *Staphylococcus aureus* (RCMB 0106), *Streptococcus pyogenes* (RCMB 0109), unicellular fungi, namely *Candid albicans* (RCMB 5002) and filamentous fungi, namely *Aspergillus , fumigatus* (RCMB 2003) and *Penicllium expansum* (RCMB 1006) were used as test organisms (RCMB: The Regional Center for Mycology and Biotechnology, Al-azhar University Nasser City, Cairo, Egypt).

### Antimicrobial screening

For antimicrobial screening the well known disc diffusion and agar well diffusion methods were used, together with the determination of minimum inhibitory concentration (MIC), see Duguid et al., Microbial Infections, The English Language Book Society, Churchill Livingston, Edingburgh, London, 1978; vol. 1, page 110.

Disc diffusion method: The agar disc diffusion method is employed for the determination of antimicrobial activities of the extracts of the investigated fungal species. Sterilized paper discs (Whatman No. 1) (0.6 mm in diameter) were separately soaked in the extracts and transferred onto the surface of growth media seeded with the test organism. After incubation period, following suitable conditions for the test organism, the diameter of the inhibition zone around the discs was measured in millimeters. The obtained result was the mean of three experiments.

Agar well diffusion method: On the other hand, the agar well diffusion method was employed for determination of the minimum inhibitory concentration (MIC). The test organisms were separately seeded in the agar medium. The wells (10 mm in diameter) were cut from the agar and 0.1 of extract solution (different concentrations) was transferred into them. After incubation period, following suitable conditions for the test organism, the diameter of the inhibition zone around the discs was measured in millimeters. The obtained result was the mean of three experiments.

### 2. Pharmacological Activities

### Preparation of fungal extract:

The methanol extracts of *C*. *blakesleeana, C. elegans* and *C*. *homothallica* were suspended individually in sterile normal saline (0.9 % NaCl) with the aid of few drops of Tween 80 immediately before use. The concentration of the tested extracts was 10%.
**Animals**: Swiss albino mice (20-25 g), male Sprague-Dawley rats (160-180 g) and male guinea pigs (240- 260 g) were employed in the study. The animals were left for 2 weeks at room conditions to ensure stabilization before use. They were maintained on standard pellet diet and water *ad libitum* throughout the experiment.
a) **Acute Toxicity**: Mice were divided into groups of 6 and the methanol extracts of *C*. *blakesleeana, C. elegans* and *C*. *homothallica* were administered orally in increasing doses. After 24 h, the LD₅₀ was calculated for each extract according to Ghosh, Fundamentals of Experimental Pharmacology, 2nd edition, Calcutta: Scientific Book Agency, (1984), pages 154-157.
b) **Sub-chronic Toxicity**: Twenty four male Sprague-Dawley rats were randomly divided into 4 equal groups. Rats of the 1^{st} group were given a vehicle (1% v/v Tween 80) in a dose of 5 ml kg⁻¹ and left as normal control. Rats of the 2^{nd}, 3^{rd}, and 4^{th} groups were administered the methanol extracts of *C*. *blakesleeana, C. elegans* and *C*. *homothallica,* respectively in a dose of 200 mg kg⁻¹. All medications were administered orally via the aid of an oral tube for 35 days. The animals were observed for signs of abnormalities throughout the experiment. At the end of the experimental period, blood samples (2 mL) were collected from the retro-orbital venous plexus of each rat (under ether anesthesia) into clean centrifuge tubes. The blood samples were allowed to clot at room temperature for 30 min after which they were centrifuged at 10,000 rpm for 5 minutes. Sera were separate with Pasteur pipette into sterile serum sample tubes and used for biochemical assay.
   Measurement of liver and kidney function markers:
   Liver functions were evaluated by measuring the serum activity of aspartate aminotransferase (AST) and alanine aminotransferase (ALT) following the method of Reitman and Frankel, Am. J. Clin. Path., 28: 55-60, alkaline phosphatase enzyme (ALP) by the method of Babson et al., Clinical Chemistry, 12: 482-490. Serum levels of total bilirubin, total proteins and albumin were also assayed. Globulin was obtained from the difference between total proteins and albumin. Serum concentrations of uric acid, urea and creatinine were determined colormetrically as measures of kidney functions. All measurements were carried out by protocols well known in the art.
c) **Wound Healing Activity**: The excision wound model was used to monitor wound contraction and wound closure time. Four groups of male guinea pigs were used in the experiment with 6 animals in each group. At the beginning of the experiment, the dorsal skin of each guinea pig was shaved with an electric clipper. After 24 hours, all animals were anesthetized by diethyl ether and the shaved areas were sterilized with 70% alcoholic solution. A predetermined dorsal area (15 mm x 20 mm) was inflicted by cutting away a full thickness of skin (2 mm depth) using toothed forceps, scalpel and pointed scissors. A fresh surgical blade was used for the perpendicular cut in each animal and tension of skin was kept constant during the procedure. Local infection with *Staphylococcus aureus* was introduced into wounds using 0.5 mL of 10⁸ bacteria mL⁻¹ inoculums. The wounds were left undressed to the open environment and the animals were kept individually in separate cages.
   **Wounds treatment**: Wounds were air exposed and treatment began 24 h after injury. Wounds of the control animals were treated with the vehicle (1% v/v Tween 80). Guinea pigs of the second and third group were treated with total alcohol extract of *C*. *elegans* and the isolated compound (adenosine) in a concentration of mg mL⁻¹, while animals of the fourth group were medicated with latamoxef (moxalactam)^{®} in a concentration of 30 µg mL⁻¹ (Reference group). The vehicle, extract, and reference drug were applied topically on the wound surface once a day for 18 days. Five mL of each tested material was applied slowly, using an insulin syringe, from the central point extending outside the wound area to ensure inclusion of the edges. Swabs were taken on day 5 to confirm the presence of the expected organisms in the pus.
   **Assessment of wound healing**: The wound areas were measured while the animals were under anaesthesia on days 0, 4, 7, 10, 14, and 18 post-wounding by tracing the wound areas onto a transparent tracing paper. Wound contraction (%) was determined using the formula of Wound contraction (%) = [(WD0 - WDt) / WD0] 100, where: WD0 = the wound diameter on day zero, WDt = the wound diameter on day t.
   **Statistical Analysis**: All the values were expressed as mean ± S. E. M. Statistical analysis was done by using SPSS 10. Statistical significance of differences between two means was assessed by unpaired Student's 't' test. Differences at p<0.05, 0.01, and 0.001 were considered statistically significant.

### Results

### 1) Antimicrobial Activity

The extracts of *Cunninghamella blakesleeana, C.elegans* and *C.homothallica* were screened for their antimicrobial activities against representatives of Gram positive, Gram negative bacteria, unicellular and filamentous fungi.

Each fungal species was grown on malt extract media at 28±2 °C for 14 days. The metabolites of the fungal species were screened for antimicrobial activity. The inhibition zones around each disc were measured, in millimeter, and recorded (Tables 1).

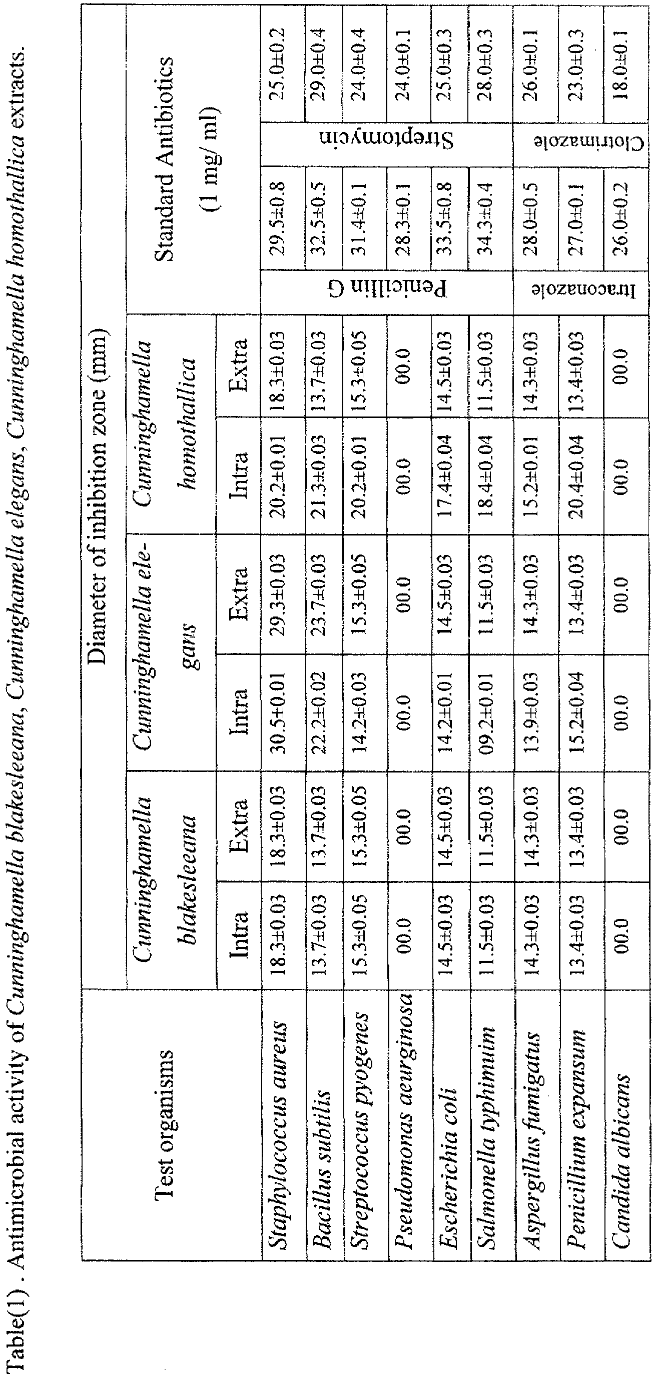

The obtained results revealed that the extract of all the investigated fungal species showed no activity against the tested unicellular fungi. However, there was a significant activity against Gram negative and/or Gram positive bacteria.

The extracts of the investigated fungal species showed good activity against all the Grampositive test organisms.

The antimicrobial activity of successive extracts of *Cunninghamella elegans* were determined (Table 2). The highest activity was obtained by ethyl acetate extract against *Staphylococcus aureus* (29.2±0.08), followed by ether extract (26.2±0.08). On the other hand, lowest activity was obtained by butanol extract (2.4±0.1), followed by ethyl acetate extract (3.4±0.5) against *Streptococcus pyogenes.*

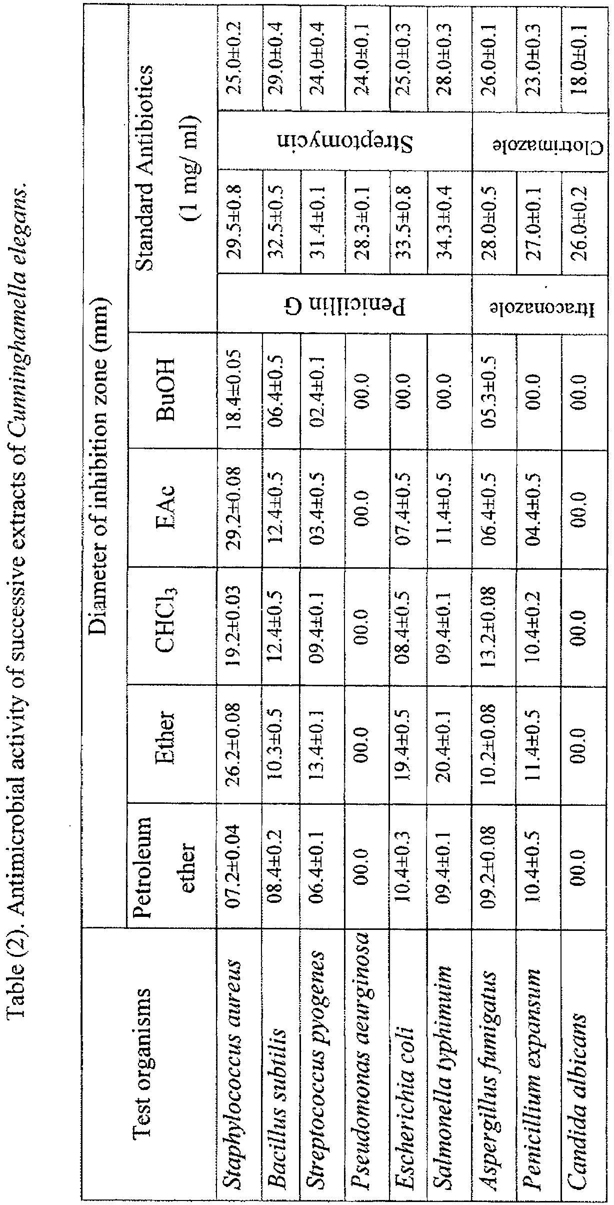

The antimicrobial activity of compounds isolated from *Cunninghamella elegans* was also determined (Table 3). Substantially, only the test organism *Staphylococcus aureus* was affected by the isolated compounds. However, the highest effect was obtained by compound 4 (adenosine) (30.0±0.01).

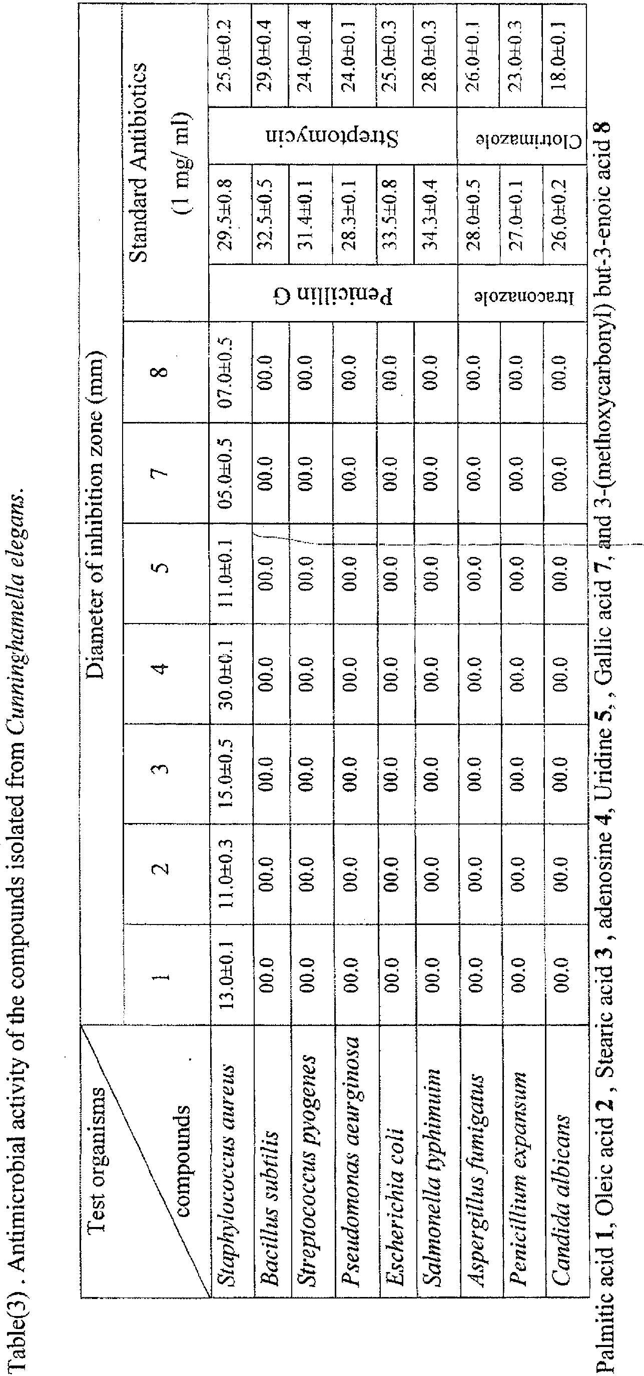

The minimum inhibitory concentration (MIC) of *Cunninghamella blakesleeana, C.elegans, C.homothallica* and successive extracts and isolated compounds of *Cunninghamella elegans* against *Staphylococcus aureus* was determined.

**Table(4) . Minimum inhibitory concentration of extracts and isolated compounds of Cunninghamella elegans against Staphylococcus aureus.**

| Extracts & compounds | | Minimum inhibitory concentration (µg/ml) |
|---|---|---|
| *Cunninghamella blakesleeana* | | |
| | - Total extract | 420 |

| *Cunninghamella homothallica* | | |
|---|---|---|
| | - Total extract | 375 |

| *Cunninghamella elegans* | | |
|---|---|---|
| | - Total extract | 250 |
| | - Ether extract | 100 |
| | - Chloroform extract | 260 |
| | - Ethyl acetate extract | 050 |
| | - Butanol extract | 200 |
| | - Compound 4 | 020 |
| | - Compound 5 | 150 |
| | - Compound 7 | 130 |
| | - Compound 8 | 210 |

| Standard antibiotics | | |
|---|---|---|
| | - Vancomycin | 0.75 |
| | - Gentamicin | 0.35 |

| | | |
|---|---|---|
| Compounds: adenosine **4**, Uridine **5**, , Gallic acid **7**, and 3-(methoxycarbonyl) but-3-enoic acid **8** | | |

Minimum inhibitory concentration (MIC): The lowest concentration of an antimicrobial agent that will inhibit a visible growth of a microorganism after overnight incubation.

The activity of extract of *Cunninghamella elegans* was (250 µg/ml), C. *homothallica* (375 µg/ml), and *Cunninghamella blakesleeana* (420 µg/ml).

On the other hand, the best activity was obtained by the isolated compound 4 (adenosine) 20 µg/ml, followed by ethyl acetate extract (50 µg/ml), and ether extract (100 µg/ml).

### 2) Pharmacological activities:

a) **Acute Toxicity Study**: All rats treated with the methanol extracts of *C*. *blakesleeana, C. elegans* and *C*. *homothallica* in doses up to 5000 mg kg⁻¹ were alive during the 24 hours of observation. The animals did not show visible signs of acute toxicity, therefore it suggested that the values of median lethal dose (LD₅₀) of the tested extracts were higher than 5000 mg kg⁻¹. The tested extracts of *C. blakesleeana, C. elegans* and *C. homothallica* are considered to be highly safe since substances possessing LD₅₀ higher than 50 mg kg⁻¹ are non toxic.
b) **Sub-chronic toxicity:** Rats received the vehicle and the methanol extracts of *C*. *blakesleeana, C. elegans* and *C*. *homothallica* by oral gavage for 35 days survived until the endpoint of the experiment. No clinical signs were observed in rats of all groups over the duration of the experiment. Oral dosing of the methanol extracts of *C*. *blaksleeana, C. elegans* and *C*. *homothallica* in a dose of 200 mg kg⁻¹ for 35 days to rats did not show any significant effect on the activity of AST and ALT in their sera as compared to control. The serum transaminase level is the most widely used as a measure of hepatocyte injury, due to its ease of measurement and high degree of sensitivity. It is useful for the detection of early damage of hepatic tissue. Since the activity of AST and ALT are specific assayable liver enzymes, their normal levels in serum of experimental groups of rats treated for 35 days means that the methanol extracts of *C*. *blakesleeana, C. elegans* and *C*. *homothallica* are not hepatotoxic. No significant changes in the mean values of ALP, total bilirubin, total protein, albumin and globulin were found in serum of rats following 35 days of extracts administration when compared with the control animals.
   In the present study, no significant change in the mean values of uric acid, urea and creatinine was found in serum of rats following 35 days of extracts administration. Urea and creatinine are the most sensitive biochemical markers employed in the diagnosis of renal damage. In kidney damage, there will be retention of urea and creatinine in the blood, therefore marked increase in serum urea and creatinine are indications of functional damage to the kidney. The methanol extracts of *C*. *blakesleeana, C. elegans* and *C*. *homothallica,* by this indicator are therefore, not nephrotoxic in rats.
c) Wound Healing Activity: Wounds occur when the continuity of the skin or mucous membrane is broken. Initially all guinea pigs had wounds of similar areas (300 mm2) which were accomplished by using stencil. Wound healing involves regeneration of specialized cells by proliferation of surviving cells and connective tissue response characterized by the formation of granulation tissue. The results of present study revealed that the topical application of methanol extract of C. elegans on the experimentally excised wound surface in a concentration of 5 mg mL-1 accelerated the wound healing process (Table 8). This shows that the isolated compound 4 (adenosine) is effective in healing excision type of wounds such as abrasions. The wound area in compound 4-medicated group was reduced to 83.4 ± 3.16 mm2 on day 10, 22.4 ± 1.15 mm2 on day 14, and 0.0 mm2 (complete closure) on day 18. The corresponding figures for the control animals were 152.5 ± 6.38 mm2 (day 10), 95.3 ± 3.47 mm2 (day 14) and 32.4 ± 1.66 mm2 (day 18). The figures for the reference drug; latamoxef were 42.6 ± 1.85 mm2 (day 10), and 0,0 mm2 (day 14). A remarkable increase in the percentage of wound contraction was observed in compound 4- treated guinea pigs as compared with the control. Topical application of compound 4 showed a 76.50% contraction on Day 14, which was close to the contraction value of the reference drug; latamoxef (100%). Complete wound contraction took place in compound 4- medicated animals; 4 days after those treated with the reference drug. A drug to be used for effective wound healing should be able to clear the wound by the 19th day after infliction, see Esimone et al., J. Pharmaceut. Allied Sci. 3 (1), 294-299 (2005). No mortality was noticed amongst the animals in all the three groups.

**Table (8): Effect of topical application of total alcohol extract, compound 1 (5 mg mL⁻¹) and latamoxef (30 µg mL⁻¹) on the wound area of Staphylococcus aureus infected wounds in guinea pig. (n=6).**

| **Treatment** | **Wound area (mm²) on day** | | | | | |
|---|---|---|---|---|---|---|
| | **0** | **4** | **7** | **10** | **14** | **18** |
| **Control** | 300 ± 0.0 | 264.6 ± 9.55 | 205.2 ± 9.45 | 152.5± 6.38 | 95.3 ± 3.47 | 32.4 ± 1.66 |
| **Total alcohol extract** | 300 ± 0.0 | 235.8 ± 9.14* | 165.6 ± 6.88** | 83.4 ± 3.16*** | 22.4 ± 1.15*** | 0.0 ± 0.0 |
| **compound 1 (adenosine)** | 300 0.0 | 241.2 ± 4.23* | 168.1 ± 2.18** | 85.6 t 4.13*** | 23.3 ± 1.11*** | 0.0 ± 0.0 |
| **Latamoxef** | 300 ± 0.0 | 219.4 ± 9.37** | 146.4 ± 5.60*** | 42.6 ± 1.85*** | 0.0 ± 0.0 | - |

| | | | | | | |
|---|---|---|---|---|---|---|
| Statistically significant at: *P<0.05 **P<0.01 ***P<0.001 | | | | | | |

Conclusively, the compound 4 (adenosine) is safe, showed antimicrobial activity against some microbes and exhibited good wound healing effect comparable to those of latamoxef, a standard antibiotic used in wound healing.

Table 8 demonstrates the results for utilizing adenosine only. As adenosine is a component of the alcoholic extract of fungi of the genus of *Cunninghamella,* similar results are also obtained utilizing an alcoholic extract according to the invention.

## Claims

1. Alcoholic extract obtained by a method for preparing an alcoholic extract, comprising the step of extracting fungi of the genus *Cunninghamella* with an alcohol, wherein the extraction is carried out under reflux, for use in therapy as antibiotic and/or antimicrobial agent.

2. Alcoholic extract for use according to claim 1, wherein the alcohol is methanol or ethanol, or mixtures thereof.

3. Alcoholic extract for use according to claim 1 or 2, wherein the fungus is *Cunninghamella blakesleeana, Cunninghamella elegans, Cunninghamella homothallica* or mixtures thereof.

4. Alcoholic extract for use according to any of the preceding claims, wherein the alcoholic extract contains at least one compound selected from the group of palmitic acid, oleic acid, stearic acid, 2-(6-amino-9H-purin-9-yl)-5-(hydroxymethyl)oxolane-3,4-diol, pyrimidine-2,4-dion,1-ribose, pyrimidine-2,4-dion, 3,4,5-trihydroxy-benzoic acid, 3-(methoxycarbonyl)-but-3-enoic acid and mixtures thereof.

5. Alcoholic extract for use according to any of the claims 1-4 for the treatment of gram negative bacteria, gram positive bacteria, or filamentous fungi, preferably *Aspergillus fumigatus, Penicillium expansum, Staphylococcus aureus, Bacillus subtilis, Streptococcus pyogenes, Escherichia coli* and *Salmonella typhimuium.*

6. Adenosine for use in therapy as antibiotic agent.

## Patentansprüche

1. Alkoholisches Extrakt erhalten durch ein Verfahren zum Herstellen eines alkoholischen Extraktes, umfassend den Schritt der Extrahierung von Pilzen der Gattung *Cunninghamella* mit einem Alkohol, wobei die Extraktion unter Refluxieren durchgeführt wird, für die therapeutische Verwendung als antibiotischer und/oder antimikrobieller Wirkstoff.

2. Alkoholisches Extrakt für die Verwendung nach Anspruch 1, wobei der Alkohol Methanol oder Ethanol oder ein Gemisch dieser ist.

3. Alkoholisches Extrakt für die Verwendung nach Anspruch 1 oder 2, wobei der Pilz *Cunninghamella blakesleena, Cunninghamella elegans, Cunninghamella homothallica* oder ein Gemisch dieser ist.

4. Alkoholisches Extrakt für die Verwendung nach einem der vorangegangenen Ansprüche, wobei das alkoholische Extrakt mindestens eine Verbindung ausgewählt aus der Gruppe aus Palmitinsäure, Ölsäure, Stearinsäure, 2-(6-Amino-9H-purin-9-yl)-5-(hydroxymethyl)oxolan-3,4-diol, Pyrimidin-2,4-dion-1-ribose, Pyrimidin-2,4-dion, 3,4,5-Trihydroxybenzoesäure, 3-(Methoxycarbonyl)-but-3-ensäure und Gemische dieser enthält.

5. Alkoholisches Extrakt für die Verwendung nach einem der Ansprüche 1-4 für die Behandlung von gramnegativen Bakterien, grampositiven Bakterien oder Fadenpilzen, vorzugsweise *Aspergillus fumigatus, Penicillium expansum, Staphylococcus aureus, Bacillus subtilis, Streptococcus pyrogenes, Escherichia coli* und *Salmonella typhimuium.*

6. Adenosin für die therapeutische Verwendung als antibiotischer Wirkstoff.

## Revendications

1. Extrait alcoolique obtenu par un procédé de préparation d'un extrait alcoolique, comprenant l'étape d'extraction de champignons du genre *Cunninghamella* avec un alcool, dans lequel l'extraction est réalisée sous reflux, pour son utilisation en thérapie comme antibiotique et/ou agent antimicrobien.

2. Extrait alcoolique pour une utilisation selon la revendication 1, dans lequel l'alcool est le méthanol ou l'éthanol, ou des mélanges de ceux-ci.

3. Extrait alcoolique pour une utilisation selon la revendication 1 ou 2, dans lequel le champignon est *Cunninghamella blakesleeana, Cunninghamella elegans, Cunninghamella homothallica* ou des mélanges de ceux-ci.

4. Extrait alcoolique pour une utilisation selon l'une quelconque des revendications précédentes, dans lequel l'extrait alcoolique contient au moins un composé choisi dans le groupe de l'acide palmitique, l'acide oléique, l'acide stéarique, le 2-(6-amino-9H-purin-9-yl)-5-(hydroxyméthyl)oxolane-3,4-diol, la pyrimidine-2,4-dione, le 1-ribose, la pyrimidine-2,4-dione, l'acide 3,4,5-trihydroxy-benzoïque, l'acide 3-(méthoxycarbonyl)-but-3-énoïque et des mélanges de ceux-ci.

5. Extrait alcoolique pour une utilisation selon l'une quelconque des revendications 1-4 pour le traitement de bactéries gram négatives, de bactéries gram positives, ou de champignons filamenteux, de préférence *d'Aspergillus fumigatus,* de *Penicillium expansum,* de *Staphylococcus aureus,* de *Bacillus subtilis,* de *Streptococcus pyogenes, d' Escherichia coli* et de *Salmonella typhimuium .*

6. Adénosine pour une utilisation en thérapie comme agent antibiotique.
